# EUROPEAN PATENT SPECIFICATION

(11) **EP 1 898 812 B1**
(45) Date of publication and mention of the grant of the patent: **27.08.2014**
(21) Application number: 06773860.9
(22) Date of filing: 21.06.2006
(51) Int. Cl.: A61B 17/56, A61B 17/16, A61B 17/17, A61B 17/04

(54) **ARTHROSCOPIC APPARATUS FOR TISSUE ATTACHMENT TO BONE**
ARTHROSKOPISCHES GERÄT ZUR GEWEBEANHEFTUNG AUF KNOCHEN
APPAREIL ARTHROSCOPIQUE POUR LA FIXATION DE TISSUS SUR DES OS

(30) Priority: 21.06.2005 US 157631
(43) Date of publication of application: 19.03.2008
(73) Proprietor: MicroAire Surgical Instruments, LLC, Charlottesville, VA 22911 (US)
(72) Inventor: Dross, Brian, Mount Pleasant, SC 29466 (US)
(74) Representative: Chapman, Paul Gilmour
(86) International application number: PCT/US2006/024523
(87) International publication number: WO 2007/002432

(56) References cited:
- EP-A- 0 081 857
- WO-A-00/67651
- DE-A- 3 411 891
- US-A- 4 541 423
- US-A- 4 744 353
- US-A- 4 941 466
- US-A- 5 330 468
- US-A- 6 059 789

## Description

### FIELD OF THE INVENTION

The invention relates to methods and devices and more specifically relates to the arthroscopic fixation of tissue to bone using sutures.

### BACKGROUND OF THE INVENTION

Invasive and open surgery methods of attachment of tissue to bone to repair tissue is known and used. An example of this method is rotator cuff surgery. In some processes, foreign objects, such as suture anchors, staples or screws, are implanted and used to connect tissue to bone.

### SUMMARY OF THE INVENTION

The present invention overcomes the invasive nature of tissue repairs by open surgical processes, and reduces the reliance on implants associated with arthroscopic repairs. The invention uses the bone constructs of the patient to attach sutures to torn or dysfunctional tissue. Suture is passed through intersecting tunnels formed in the bone. An end of the suture extends from each of the tunnels, and the ends are used to secure the tissue to the bone, such as by arthroscopic tying of the ends, and pulling the tissue against the bone.

US 5,330,468 describes a device for the arthroscopic fixation of tissue to bore in the rotator of the shoulder which comprises an arcuate drill guide and a straight drill guide each having a lumen, a handle for connecting said drill guides, and a flexible stylus which is passed through the lumen of one guide and drills a straight tunnel through the humerus where it is received by the other guide.

### DESCRIPTION OF THE DRAWINGS

FIG. 1 illustrates a rotator cuff drill guide in place with a trephine piercing a torn rotator cuff. A single stand of suture is shown passing through the drill guide, rotator cuff tissue, into and out of the humeral head, and exiting the central lumen of the trephine.
FIG. 2 illustrates three arthroscopic simple stitches, a repaired rotator cuff, and two lateral vertical portals.
FIG. 3 illustrates two arthroscopic mattress stitches where the initial suture in the center bone tunnel was used to pass two sutures.

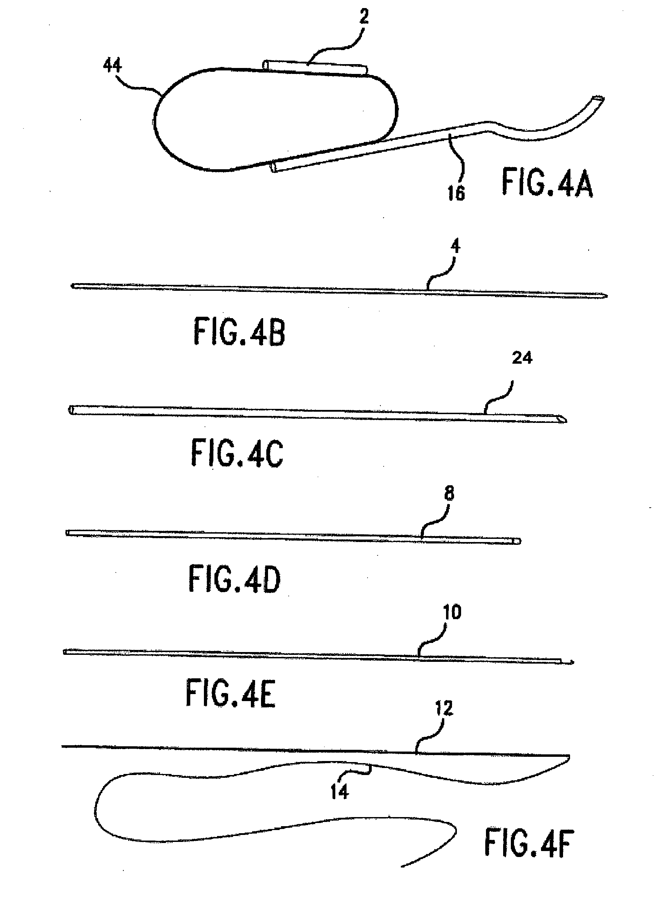

FIG. 4A is the drill guide assembly having an arcuate drill guide, a straight drill guide and a handle.
FIG. 4B is the stylus for the arcuate drill guide lumen.
FIG. 4C is the stylus for use as a trephine guide pin that will fit into the straight drill guide lumen with enough clearance for the trephine.
FIG. 4D is the arthroscopic trephine.
FIG. 4E is an offset hook probe that will pass into the trephine.
FIG. 4F is a suture stylus with suture loosely attached.

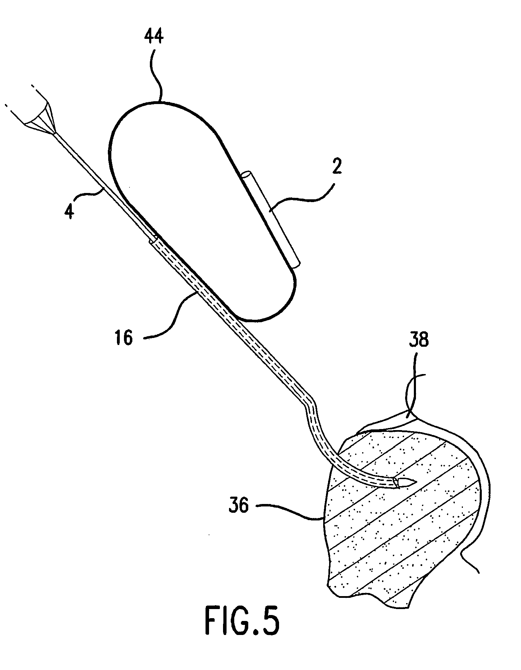

FIG. 5 illustrates insertion of the arcuate lumen rotator cuff drill guide leading with the stylus.
FIG. 6 illustrates the stylus that has been passed through the straight drill guide lumen.
FIG. 7 illustrates the trephine inserted to a calibration point that advances the tip past the bone void left by the stylus.
FIG. 8 illustrates the trephine partially retracted to a second calibration mark, the suture stylus with suture being advanced and the hook probe in a readied position.
FIG. 9 illustrates the suture lodged in the bone void left by the stylus after being left behind by the suture stylus, and the hook probe which has been passed through the trephine to capture the suture.
FIG. 10 demonstrates a device using straight, but non-parallel drill guides, which is outside the scope of the present invention.
FIG. 11 demonstrates an embodiment of the device showing an additional configuration of an arcuate drill guide.

### DETAILED DESCRIPTION OF THE PREFERRED EMBODIMENT

A preferred embodiment of the invention is demonstrated in repairing a rotator cuff. Two arthroscopic portals 30,32 are formed in the shoulder 34, such as by a scalpel. The humeral head 36 and rotator cuff tendons 38 are present. An arcuate drill guide 16 having a central lumen is inserted into one of the portals. If required, cortical bone may be removed prior to insertion of the arcuate drill guide.

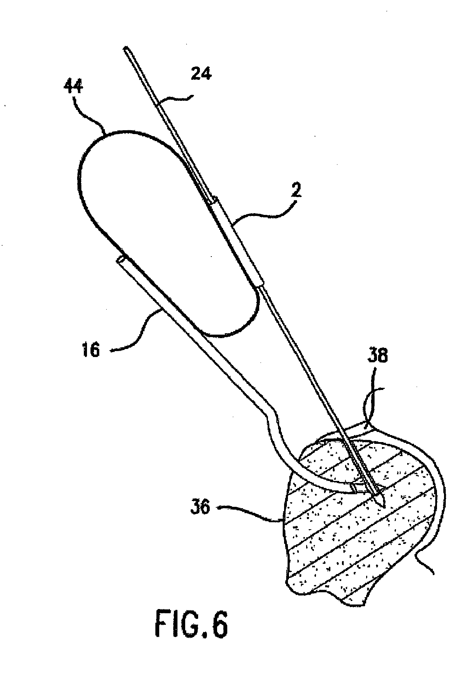

The central lumen of the arcuate drill guide 16 has a protruding flexible stylus 4 therein that is advanced into the humeral head lateral of the torn rotator cuff. The stylus 4 may be formed of nitinol. The stylus 4 may have a cutter formed in an end thereof, such as a drill or mill type cutter. In this embodiment, the forward end of the arcuate drill guide 16 is curved. Advancement of the arcuate drill guide 16 may be by manual pressure or by assisted manual force using, for example, a mallet, or by a power tool, such as a drill. The arcuate drill guide 16 forms an arcuate tunnel in the bone. After the arcuate drill guide 16 is fully advanced, the drill guide stylus 4 is withdrawn, leaving a small void in the bone that is present beyond the leading edge of the arcuate drill guide. FIG. 6.

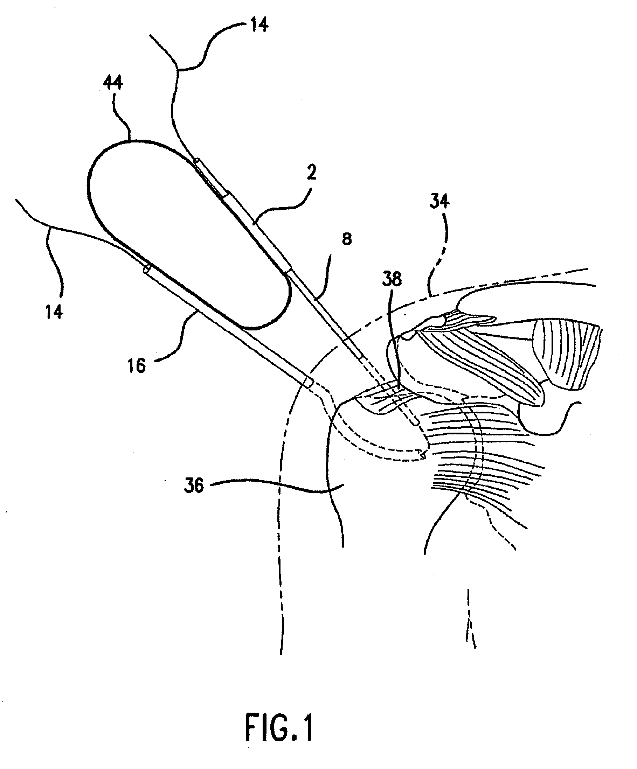

As shown in FIG. 1, straight drill guide 2 is placed through the other portal. The straight drill guide 2 has a lumen therein. A stylus 24 is positioned within this lumen. The stylus 24 may be formed of nitinol. Sufficient space is present within the lumen for placement of the trephine 8, so that the stylus 24 has a sloppy fit within the drill guide. The stylus 24 pierces the tissue.

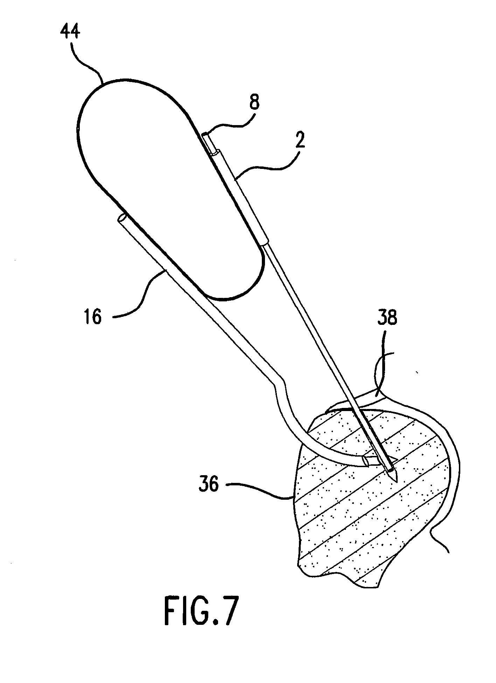

The trephine 8 is inserted into the lumen of the straight drill guide 2. The trephine 8 has a larger diameter than the stylus 24, but will rotate within the lumen. The trephine 8 enlarges the tunnel, and is moved past the arcuate shaped tunnel formed using the arcuate drill guide 16. FIG. 7.

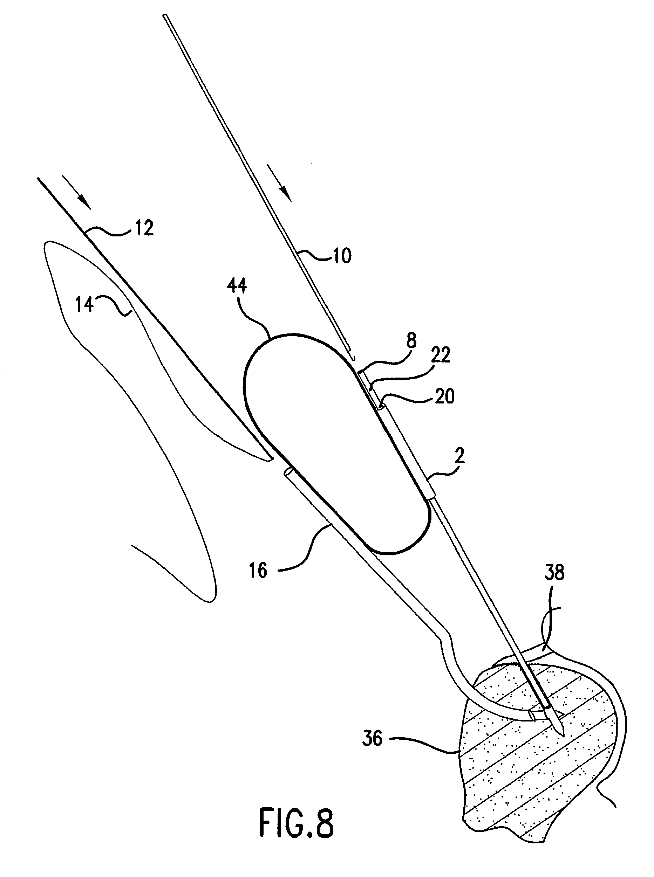

The trephine 8 is retracted. As shown in FIG. 8 the trephine 8 may have calibration marks 20, 22 to indicate the depth of insertion and retraction of the trephine 8. The bone tunnels intersect as shown.

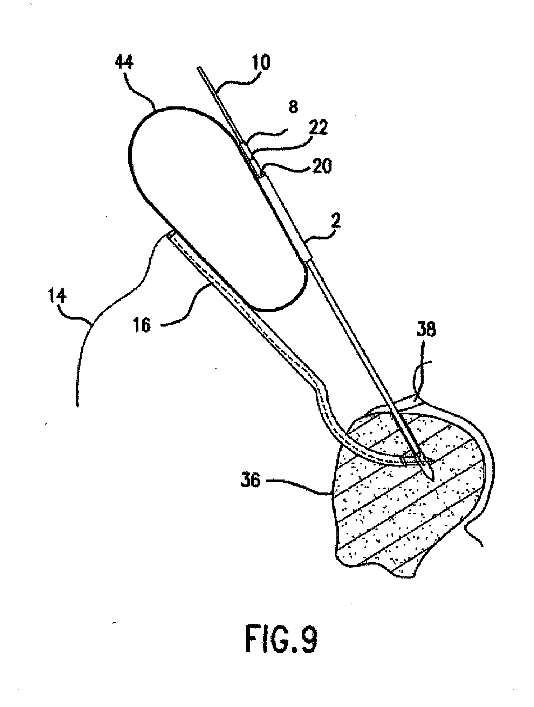

With the trephine 8 in place, but with the styli 4,24 removed from the drill guides 2,16, one or more strands of suture 14 are passed through the lumens of the drill guides 2,16, through a reapproximated rotator cuff tear, and through the two converging bone tunnels. The suture also passes through the humeral head (bone), and exits the central lumen of the trephine. The suture is advanced through the arcuate drill guide 16 by the suture stylus 12. The hook probe 10 is inserted through the lumen of the trephine 8 to hook the suture advanced by the suture stylus at approximately the intersection of the tunnels. FIG. 9.

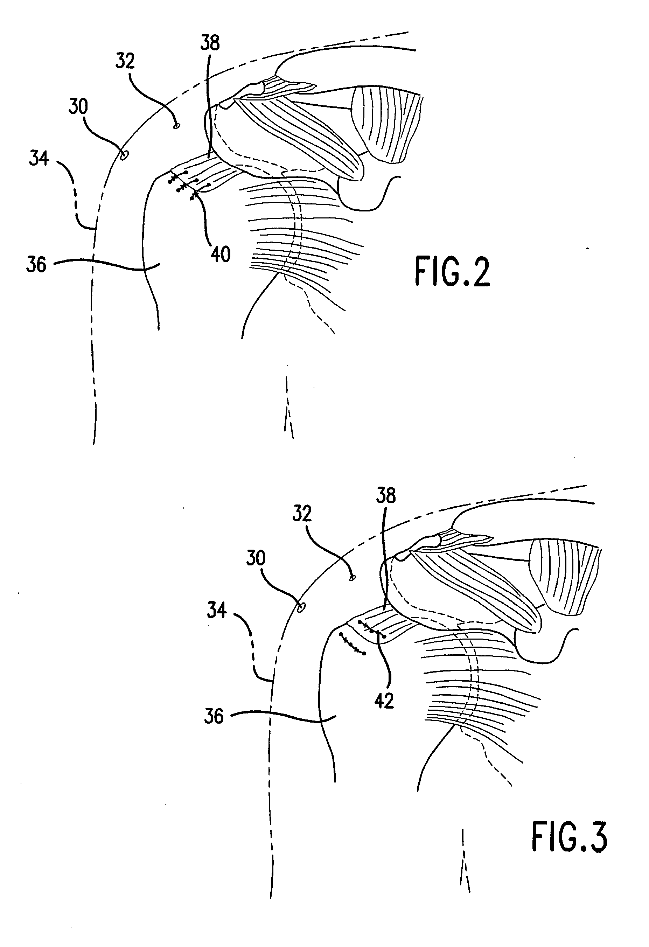

Removal of the drill guides 2,16 leaves the suture in place for tying. Multiple suture passes allow for tying of the suture material. For example, three (3) suture passes allow tying three (3) simple stitches 40 as shown in FIG. 2.

FIG. 3 shows two arthroscopic mattress stitches 42 where the initial suture in the center bone tunnel was used to pass two sutures. The two sutures were tied twice with their adjacent sutures to form mattress stitches. Alternatively, the outside suture strands could have been used to pull the corresponding central suture into the outside tunnel, resulting in one less knot left in the patient, and the opportunity to use a sliding knot.

The arcuate drill guide 16 and the straight drill guide 2 may be connected by a handle 44. The handle positions the relative angles of the drill guides 2,16 for forming the tunnels as described. The drill guides 2,16 are positioned by the handle 44 so that intersecting tunnels are formed as disclosed herein. The handle 44 may also be used to receive and transfer a force for advancing the drill guides, such as by striking the handle with a mallet.

Benefits of the present invention over the use of suture anchors include the introduction of minimal foreign material in the patient, a larger "healing footprint" (which is variable with the distance between lumens) and the use of lumens as injection ports for plate rich/poor blood/growth factors. This method of arthroscopic bone/suture tunnel creation also has applications in shoulder laberal repair and posterior cruciate ligament and anterior cruciate ligament repair, without, or at least reducing, the requirement of suture anchors, staples or screws. The geometry of the apparatus relates to an arthroscopic creation of bone tunnels and simultaneous suture passing to repair a torn or partially torn rotator cuff.

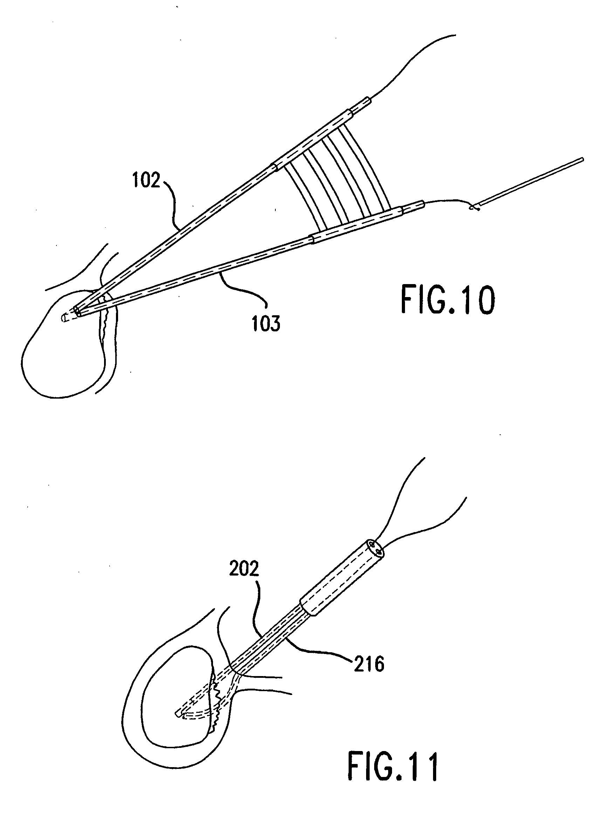

FIG. 10 shows the method of arthroscopic attachment of tissue to bone where different drill guide configurations are used to address the anatomic structure of the genohumeral joint, which are different that rotator cuff repair. FIG. 10 shows parallel drill guide lumens 102,103 that are useful for superior labrum deficiencies or tears.

FIG. 11 shows a drill guide that is similar to the rotator cuff guide, having one arcuate lumen 216 and one straight lumen 202 but having a different converging angle for inferior laberal repair. Aside from these differences in the apparatus, the method of arthroscopic securing tissue to the glenoid is the same as described for attaching the rotator cuff to the humeral head.

## Claims

1. A device for the arthroscopic fixation of tissue to bone in the rotator of the shoulder, comprising:
an arcuate drill guide (16) having a central lumen that has a protruding flexible first stylus (4) for advancement into the humeral head lateral of the rotator cuff to form a first tunnel, wherein the guide or the first stylus can form the first tunnel;
a straight drill guide (2) having a lumen (6) positioned therein and a second stylus (24) positioned within said lumen 6 capable of forming a second tunnel; and
a handle (44) for connecting said drill guides (2, 16) at the relative angles of the drill guides for forming tunnels so that intersecting/bisecting tunnels are formed.

2. The device of claim 1, further comprising a trephine (8) which can be inserted into the lumen of the straight drill guide (2) to enlarge said second tunnel.

3. The device of claim 1, further comprising a suture stylus (12).

4. The device of claim 2, wherein the trephine (8) comprises a lumen through which a hook (10) is inserted.

5. The device of claims 1 - 4, wherein the suture stylus (12) is formed of nitinol.

6. The device of claim 1 - 5, wherein the first stylus (4) has a cutter formed in an end thereof.

7. The device of claims 1 - 6, wherein the handle (44) receives and transfers a force advancing the drill guides (2, 16).

## Patentansprüche

1. Vorrichtung für die arthroskopische Befestigung von Gewebe am Knochen im Rotator der Schulter, die aufweist:
eine bogenförmige Bohrerführung (16) mit einem zentralen Lumen, die einen vorstehenden flexiblen ersten Taststift (4) für die Beförderung in den humeralen Kopf seitlich der Rotatorenmanschette aufweist, um einen ersten Tunnel zu bilden, wobei die Führung oder der erste Taststift den ersten Tunnel bilden kann;
eine geradlinige Bohrerführung (2) mit einem darin positionierten Lumen (6) und einem zweiten Taststift (24), der innerhalb des Lumens (6) positioniert ist, die einen zweiten Tunnel bilden kann; und
einen Griff (44) für das Verbinden der Bohrerführungen (2, 16) unter relativen Winkeln der Bohrerführungen für das Bilden von Tunneln, so dass sich schneidende/halbierende Tunnel gebildet werden.

2. Vorrichtung nach Anspruch 1, die außerdem eine Trephine (8) aufweist, die in das Lumen der geradlinigen Bohrerführung (2) eingesetzt werden kann, um den zweiten Tunnel zu vergrößern.

3. Vorrichtung nach Anspruch 1, die außerdem einen Nahttaststift (12) aufweist.

4. Vorrichtung nach Anspruch 2, bei der die Trephine (8) ein Lumen aufweist, durch das ein Haken (10) eingesetzt wird.

5. Vorrichtung nach einem der Ansprüche 1 bis 4, bei der der Nahttaststift (12) aus Nitinol gebildet wird.

6. Vorrichtung nach Anspruch 1 bis 5, bei der der erste Taststift (4) eine Schneide aufweist, die in einem Ende davon gebildet wird.

7. Vorrichtung nach Anspruch 1 bis 6, bei der der Griff (44) eine Kraft aufnimmt und überträgt, die die Bohrerführungen (2, 16) vorwärts bewegt.

## Revendications

1. Dispositif pour la fixation arthroscopique de tissus à l'os dans le rotateur de l'épaule, comprenant :
un guide-foret arqué (16), comportant une lumière centrale comportant un premier stylet flexible en saillie (4) en vue d'une avance dans la tête humérale latérale de la coiffe du rotateur afin de former un premier tunnel, le guide ou le premier stylet pouvant former le premier tunnel ;
un guide-foret droit (2), comportant une lumière (6) qui y est positionnée, et un deuxième stylet (24) positionné dans ladite lumière (6), capable de former un deuxième tunnel ; et
une poignée (44) pour connecter lesdits guide-forets (2, 16) au niveau des angles relatifs des guide-forets afin de former des tunnels, de sorte à former des tunnels à intersection/bissection.

2. Dispositif selon la revendication 1, comprenant en outre une tréphine (8) pouvant être insérée dans la lumière du guide-foret droit (2) afin d'élargir ledit deuxième tunnel.

3. Dispositif selon la revendication 1, comprenant en outre un stylet de suture (12).

4. Dispositif selon la revendication 2, dans lequel la tréphine (8) comprend une lumière à travers laquelle un crochet (10) est inséré.

5. Dispositif selon les revendications 1 à 4, dans lequel le stylet de suture (12) est composé de nitinol.

6. Dispositif selon les revendications 1 à 5, dans lequel le premier stylus (4) comporte un élément de coupe formé dans une de ses extrémités.

7. Dispositif selon les revendications 1 à 6, dans lequel la poignée (44) reçoit et transfère une force faisant avancer les guide-forets (2, 16).
